Europäisches Patentamt

European Patent Office

Office européen des brevets

(11) Veröffentlichungsnummer: **0 400 297**
**A2**

## (12) EUROPÄISCHE PATENTANMELDUNG

(21) Anmeldenummer: 90106092.1

(51) Int. Cl.⁵: **A61B 17/22**

(22) Anmeldetag: 30.03.90

(30) Priorität: 31.05.89 DE 3917663

(43) Veröffentlichungstag der Anmeldung:
05.12.90 Patentblatt 90/49

(84) Benannte Vertragsstaaten:
**DE FR GB IT NL**

(71) Anmelder: **Richard Wolf GmbH**
**Pforzheimer Strasse 32**
**D-7134 Knittlingen(DE)**

(72) Erfinder: **Weissmüller, Johannes, Dr.**
**Am Bahndamm 6**
**D-8501 Schwaig(DE)**
Erfinder: **Ell, Christian, Dr. med.**
**Burgbergstrasse 16**
**D-8520 Erlangen(DE)**
Erfinder: **Hochberger, Jürgen, Dr.**
**Wöhrstrasse 6a**
**D-8520 Erlangen(DE)**
Erfinder: **Bonnet, Ludwig**
**Jahnstrasse 28**
**D-7134 Knittlingen(DE)**
Erfinder: **Kolb, Achim, Dipl.-Phys.**
**Turbanstrasse 2/1**
**D-7518 Bretten(DE)**

(74) Vertreter: **Wilcken, Hugo, Dr. et al**
**Patentanwälte Dr. Hugo Wilcken Dipl.-Ing.**
**Thomas Wilcken Musterbahn 1**
**D-2400 Lübeck(DE)**

(54) **Endoskop für die Laser-Lithotripsie.**

(57) Das für die Laser-Lithotripsie eingerichtete Endoskop besitzt eine Laser-Lithotripsie-Einheit (6) mit einem in den Endoskopschaft (1) einschiebbaren Führungsrohr (7). Das Rohr (7) umschließt einen Laser-Lichtleiter (8), der an seinem distalen Ende einen angeschmolzenen Kopf (13) zur Strahlenbündelung trägt. Der Laser-Lichtleiter (8) ist in dem Führungsrohr (7) axial verschiebbar und fixierbar geführt, so daß die Möglichkeit der Einstellung des Abstandes des Kopfes (13) zu dem zu zerstörenden Konkrement (16) im Sinne einer optimalen Energietransmission besteht, welche Verhältnisse während des Eingriffes dadurch erhalten bleiben, daß das distale Ende des Führungsrohres (7) der Lithotripsie-Einheit (6) mit dem Konkrement (16) in Kontakt gehalten wird.

FIG. 1

FIG. 3

## Endoskop für die Laser-Lithotripsie

Die Erfindung betrifft ein Endoskop für die Laser-Lithotripsie mit einem proximal an eine Laserlichtquelle anschließbaren Lichtleiter, der einen Endoskopkanal durchläuft und dessen distales Ende unter visueller Kontrolle mit der Endoskopoptik auf das zu zerstörende Konkrement ausrichtbar ist.

In der medizinischen Technik sind mit Laser-Einrichtungen kombinierbare Endoskope für unterschiedliche Aufgaben bekannt und kommerziell verfügbar. So gehören derartige Einrichtungen zum Stand der Technik, welcher als chirurgisches Skalpell einsetzbar sind oder mit deren Hilfe beispielsweise Blutungen durch Koagulation zum Stehen bringbar oder auch eine gezielte Bestrahlung von erkrankten Organteilen durchführbar sind. Dazu weisen die Endoskope in der Regel Einführungskanäle auf, durch die die Lasereinrichtungen hindurchführbar sind, so daß neben der Möglichkeit, Behandlungen der oben beispielhaft aufgeführten Art vorzunehmen, das Arbeitsfeld während des Behandlungsvorganges visuell kontrolliert werden kann.

So sei als Beispiel auf die DE-OS 29 45 080 hingewiesen, deren Gegenstand eine Einrichtung zur endoskopischen Laser-Bestrahlung zur Behandlung von Blasentumoren ist. Bei dieser Einrichtung findet ein Rohr Verwendung, welches ein weiteres Rohr zur Aufnahme des Endoskopes sowie weitere Kanäle umschließt, die der Aufnahme bzw. Führung von Spülflüssigkeit, einer Lichtleitfaser sowie eines Schubgestänges für die Ausrichtung des distalen Endbereiches der Lichtleitfaser dienen. Mit Hilfe des Schubgestänges läßt sich das distale Ende des Lichtleiters aus der Achse des Instrumentes herausschwenken und so eine genaue Einstellung der Laser-Strahlung auf einen Behandlungspunkt vornehmen. Desweiteren ist eine Längsverschiebbarkeit der Lichtleitfaser vorgesehen.

In Verbindung mit der beschriebenen Ausrichtbarkeit soll damit ermöglicht werden, etwaige Verschmutzungen des distalen Endes der Lichtleitfaser dadurch zu beseitigen, daß dieses Ende in die Blasenflüssigkeit vorgeschoben und in dieser Stellung hin und her geschwenkt wird. Auf diese Weise soll vermieden werden, daß die Einrichtung im Falle der Verschmutzung im distalen Endbereich zwecks Säuberung aus dem Körper des Patienten entnommen und wieder eingeführt werden muß.

Schließlich sei noch auf die Einrichtung gemäß der US-PS 3 865 113 hingewiesen, die als chirurgisches Skalpell ein setzbar ist. Die Schneidfunktion wird hier ebenfalls durch einen Laserstrahl übernommen, der ein rohrförmiges Handstück durchläuft und mittels einer Linse auf einen Fokussierpunkt fokussiert wird. Für den Einsatz der Einrichtung wird das Handstück mit einem aufsteckbaren Vorsatz versehen, der jeweils in Anpassung an die jeweilige Aufgabe gestaltet ist und ein Führungselement darstellt, das dem Chirurg ermöglicht, den Fokussierpunkt in für die optimale Durchführung des Eingriffes zweckmäßigerweise zu führen.

Bei der Laser-Lithotripsie wird ein Laserstrahl hoher Energie auf das zu zerstörende Konkrement geleitet, was üblicherweise über einen Lichtleiter erfolgt, der Bestandteil einer endoskopischen Einrichtung ist, die durch Einführen in die entsprechende Körperhöhle an das betreffende Konkrement herangeführt wird. Dabei besteht die Gefahr, daß das distale Ende des Lichtleiters mit dem zu zerstörenden Konkrement in Kontakt kommt, was zur Beschädigung dieses empfindlichen Teiles führen kann. Darüberhinaus kommt es darauf an, daß die Energietransmission auf das Konkrement in optimaler Weise und reproduzierbar erfolgt, was eine genaue Einstellbarkeit des Abstandes zwischen dem distalen Ende des Lichtleiters und dem zu zerstörenden Konkrement erfordert.

Es ist daher die Aufgabe der Erfindung, ein Endoskop des eingangs beschriebenen Aufbaues so herzurichten, daß diesen Erfordernissen genüge getan werden kann.

Diese Aufgabe wird erfindungsgemäß dadurch gelöst, daß der Lichtleiter in einem axial in den Endoskopschaft einführbaren Führungsrohr angeordnet und relativ zu diesem in Axialrichtung bewegbar und fixierbar ist, um den Abstand zwischen dem distalen, gegen das Konkrement zur Anlage bringbaren Führungsrohrende und dem sich hierzu in geringem Abstand befindlichen Lichtleiterende variabel einstellen zu können.

Gemäß einer bevorzugten Ausführungsform des erfindungsgemäßen Endoskopes ist vorgesehen, daß dessen Führungsrohr ein zum Objektiv der Optik hin offenes Fenster hat, durch das mittels der Optik die distalen Endbereiche des Führungsrohres und des Lichtleiters sichtbar sind. Dadurch ist es möglich, die Vorgänge im Bereich des Arbeitsfeldes der Einrichtung visuell zu kontrollieren.

In weiterer Ausgestaltung der Erfindung ist dabei zumindest der distale Endbereich des Lichtleiters im Führungsrohr abgestützt und axial, insbesondere koaxial im Führungsrohr geführt, wobei die den Lichtleiter führende Abstützung auf der dem Fenster gegenüberliegenden Seite des Führungsrohres vorgesehen ist und als Rampe mit einer zur Führungsrohrachse parallelen Führungsfläche ausgebildet sein kann.

Eine axiale Fixierung des Lichtleiters in der optimierten Stellung kann durch Betätigen einer proximalen Klemmeinrichtung erfolgen, über die der Lichtleiter in das Führungsrohr eingeführt wurde.

Gemäß einer weiteren Ausführungsform kann das distale Ende des Lichtleiters durch einen an diesem angeschmolzenen Kopf gebildet sein. Durch diesen vorzugsweise kugelförmig gestalteten Kopf wird eine Strahlenbündelung erreicht, wodurch vor der Stirnfläche des Lichtleiters ein Plasma erzeugt wird, das an dem Konkrement einen Druckstoß bewirkt.

Schließlich hat sich eine Ausführungsform des Endoskopes als besonders vorteilhaft erwiesen, bei der das freie Lumen zwischen Lichtleiter und Führungsrohr einen distal offenen Kanal für Spülwasser bildet, das über einen proximalen Anschluß in den Kanal einführbar und nach Austritt aus diesem Kanal vom Behandlungsort aus über einen weiteren Kanal abführbar ist, der durch das freie Lumen zwischen Führungsrohr und Optik einerseits und dem Endoskopschaft andererseits gebildet ist und in einen proximalen Anschluß des Endoskopschaftes mündet. Der besondere Vorteil dieser Anordnung liegt dabei darin, daß der Spülstrahl direkt auf den Ort der Zerstörung gerichtet ist und daher die abgesprengten Partikel sofort aus dem Einwirkbereich des Laserstrahles herausgeführt werden, welcher Vorgang die Effektivität desselben weiter verbessert.

Das erfindungsgemäße Endoskop ist nachfolgend anhand eines in der Zeichnung dargestellten Ausführungsbeispieles näher erläutert. Es zeigen:

Figur 1 ein Endoskop,eingerichtet für die Lithotripsie, in Gesamtansicht,

Figur 2 eine erfindungsgemäße, in den Endoskopschaft einführbare Laser-Lithotripsie-Einheit mit einem Laser-Lichtleiter,

Figur 3 den distalen Endbereich der Einheit nach Figur 2 im Längsschnitt und vergrößert dargestellt,

Figur 4 einen Querschnitt durch den in Figur 3 dargestellten Endbereich der Einheit längs der Schnittlinie 4,

Figur 5 einen Querschnitt gemäß Figur 4 durch eine weitere Ausführungsform des distalen Endbereiches gemäß Figur 3,

Figur 6 einen Querschnitt durch den Endoskopschaft mit eingeschobener Laser-Lithotripsie-Einheit.

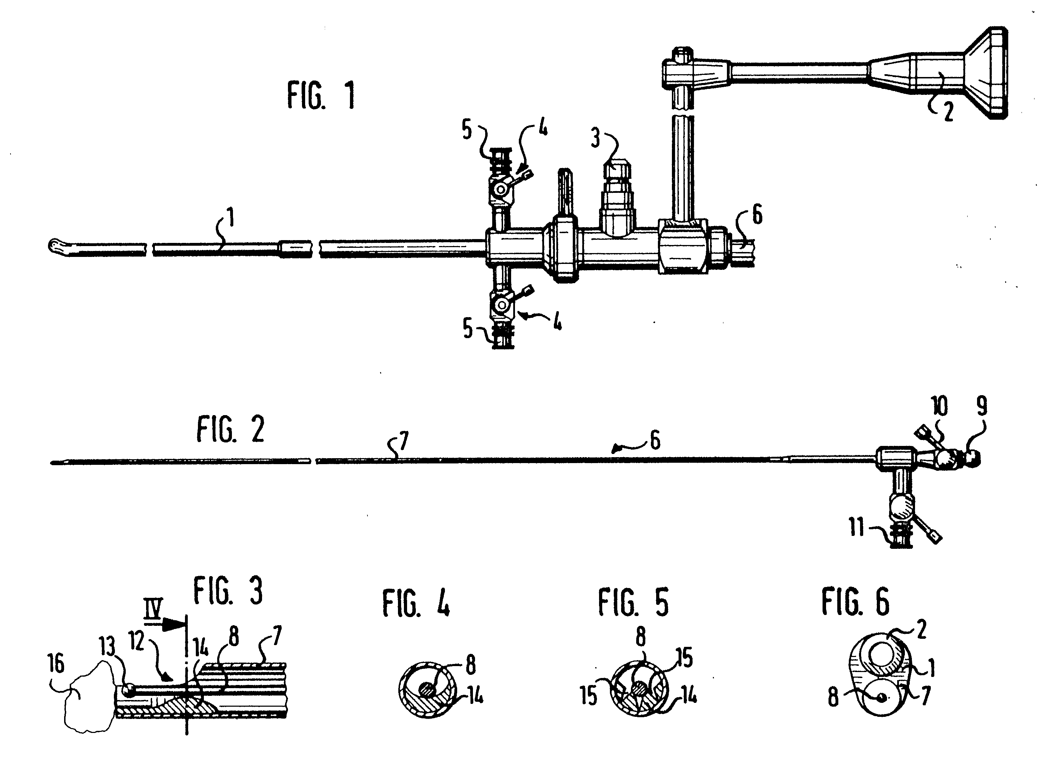

In Figur 1 ist ein Endoskop dargestellt, welches in der Praxis üblicherweise eingesetzt wird. Es umfaßt im wesentlichen einen Endoskopschaft 1, in dessen proximalem Endbereich eine Endoskopoptik 2 zur visuellen Kontrolle des Arbeitsfeldes, ein Lichtanschluß 3 für die Ausleuchtung des Arbeitsfeldes, eine Einstelleinrichtung 4 zur Steuerung des distalen Endes des Endoskopschaftes 1 in bezug auf seine Winkelabweichung von der Achse des letzteren sowie Anschlüsse 5 für die Ein- bzw. Ableitung von Spülflüssigkeit angeordnet sind. Der Endoskopschaft 1 dient der exzentrischen Aufnahme und Hindurchführung einer Laser-Lithotripsie-Einheit 6 nach Figur 2. Diese Einheit 6 umfaßt im wesentlichen ein Führungsrohr 7, welches der koaxialen Aufnahme eines Lichtleiters 8 dient. Das Führungsrohr 7 weist in seinem proximalen Endbereich einen mit dem Lichtleiter 8 in Verbindung stehenden Lichtleiter-Anschluß 9 auf sowie eine Klemmeinrichtung 10 für die axiale Fixierung des Lichtleiters 8. Darüberhinaus ist ein Spülmittelanschluß 11 in diesem Endbereich vorgesehen.

Der distale Endbereich des Führungsrohres 7 ist im Anschluß an das stirnseitige Optikausblickfenster des Endoskopes durch Entfernen eines Teiles des Rohrmantels des Führungsrohres 7 mit einem Fenster 12 versehen, so daß dieser Endbereich und das zu zerstörende Konkrement mit Hilfe der Optik 2 einstellbar ist. Damit läßt sich auch das bis in diesem Bereich vorgeschobene distale Ende des Lichtleiters 8 visuell kontrollieren, welches durch einen an diesem angeschmolzenen Kopf 13 gebildet wird. Für die zentrale Halterung und Führung des distalen Endbereiches des Lichtleiters 8 ist wie in den Figuren 4 und 5 erkennbar auf der dem Fenster gegenüberliegenden Seite des Führungsrohres 7 eine Abstützung 14 angebracht, die den Lichtleiter 8 in einer prismatischen Führung stützt. Für den Durchlaß der Spülflüssigkeit auch auf der Seite der Abstützung 14 kann diese gemäß Figur 5 mit Längsnuten 15 versehen sein.

Der bei der erfindungsgemäßen Einrichtung direkt auf die Wirkstelle gerichtete Spülflüssigkeitsstrahl ermöglicht das sofortige Ausschwemmen der abgesprengten Konkrement-Partikel aus dem Wirkungsfeld des Laserstrahles. Dabei erfolgt der Rückfluß der Spülflüssigkeit durch das relativ große freie Lumen zwischen dem Endoskopschaft 1 und dem Führungsrohr 7 der Lithotripsie-Einheit 6, so daß der Laserstrahl mit hoher Effektivität eingesetzt werden kann. Der Zufluß der Spülflüssigkeit erfolgt über den Spülmittelabschluß 11, so daß beide Anschlüsse 5 als Rücklaufanschlüsse benutzt werden können. Das Konkrement ist mit 16 bezeichnet.

## Ansprüche

1. Endoskop für die Laser-Lithotripsie mit einem proximal an eine Laserlichtquelle anschließbaren Lichtleiter, der einen Endoskopkanal durchläuft und dessen distales Ende unter visueller Kontrolle mit der Endoskopoptik auf das zu zerstörende Konkrement ausrichtbar ist, dadurch gekennzeichnet, daß der Lichtleiter (8) in einem axial in den Endoskopschaft (1) einführbaren Führungsrohr (7) angeordnet und relativ zu diesem in Axialrichtung bewegbar und fixierbar ist, um den Abstand zwischen dem distalen., gegen das Konkrement (16) zur Anlage bringbaren Führungsrohrende und dem sich hierzu in geringem Abstand befindlichen Lichtleiterende beliebig einstellen zu können.

2. Endoskop nach Anspruch 1, dadurch gekennzeichnet, daß das Führungsrohr (7) ein zum Objektiv der Optik (2) hin offenes Fenster (12) hat, durch das mittels der Optik die distalen Endbereiche des Führungsrohres (7) und des Lichtleiters (8) sichtbar sind.

3. Endoskop nach einem der Ansprüche 1 und 2, dadurch gekennzeichnet, daß zumindest der distale Endbereich des Lichtleiters (8) im Führungsrohr (7) abgestützt und axial, insbesondere koaxial, im Führungsrohr (7) geführt ist.

4. Endoskop nach Anspruch 3, dadurch gekennzeichnet, daß die den Lichtleiter (8) führende Abstützung (14) auf der dem Fenster (12) gegenüberliegenden Seite des Führungsrohres (7) vorgesehen ist.

5. Endoskop nach den Ansprüchen 3 und 4, dadurch gekennzeichnet, daß die Abstützung (14) als Rampe mit einer zur Führungsrohrachse parallelen Führungsfläche ausgebildet ist.

6. Endoskop nach einem der Ansprüche 1 bis 5, dadurch gekennzeichnet, daß der Lichtleiter (8) über eine proximale Klemmeinrichtung (10) in das Führungsrohr (7) einführbar und durch Betätigung der Klemmeinrichtung (10) fixierbar ist.

7. Endoskop nach einem der Ansprüche 1 bis 6, dadurch gekennzeichnet, daß das distale Ende des Lichtleiters (8) durch einen an diesen angeschmolzenen Kopf (13) gebildet ist.

8. Endoskop nach einem der Ansprüche 1 bis 7, dadurch gekennzeichnet, daß das freie Lumen zwischen Lichtleiter (8) und Führungsrohr (7) einen distal offenen Kanal für Spülwasser bildet, das über einen proximalen Anschluß (11) in den Kanal einführbar und nach Austritt aus diesem Kanal vom Behandlungsort aus über einen weiteren Kanal abführbar ist, der durch das freie Lumen zwischen Führungsrohr (7) und Optik (2) einerseits und dem Endoskopschaft (1) andererseits gebildet ist und in einen proximalen Anschluß (5) des Endoskopschaftes mündet.

FIG. 1

FIG. 2

FIG. 3

FIG. 4

FIG. 5

FIG. 6

EP 0 400 297 A2